(19) Europäisches Patentamt
European Patent Office
Office européen des brevets

(11) Numéro de publication: **0 284 523 B1**

(12) # FASCICULE DE BREVET EUROPEEN

(45) Date de publication du fascicule du brevet: **11.07.90**

(51) Int. Cl.⁵: **C07C 205/22**

(21) Numéro de dépôt: **88420079.1**

(22) Date de dépôt: **03.03.88**

(54) **Procédé de chloration de nitrophénols.**

(30) Priorité: **05.03.87 FR 8703209**

(43) Date de publication de la demande: **28.09.88 Bulletin 88/39**

(45) Mention de la délivrance du brevet: **11.07.90 Bulletin 90/28**

(84) Etats contractants désignés: **AT BE CH DE ES GB GR IT LI LU NL SE**

(56) Documents cités: **DE-A- 1 046 061**

(73) Titulaire: **RHONE-POULENC CHIMIE, 25, quai Paul Doumer, F-92408 Courbevoie Cédex(FR)**

(72) Inventeur: **Desmurs, Jean-Roger, La Jonquière Route de Ternay Communay, F-69360 St-Symphorien D'Ozon(FR)**
Inventeur: **Jouve, Isabelle, 30, rue Léon Fabre, F-69100 Villeurbanne(FR)**

(74) Mandataire: **Vignally, Noel et al, Rhône-Poulenc Interservices Service Brevets Chimie Centre de Recherches des Carrières B.P. 62, F-69192 Saint-Fons Cédex(FR)**

EP 0 284 523 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne la chloration de nitrophénols par le chlore gazeux.

Les chloronitrophénols sont des composés connus et le dichloro-2,6 nitro-4 phénol par exemple, est un intermédiaire chimique, servant en agrochimie après hydrogénation en amino-4 dichloro-2,6 phénol, ou comme intermédiaire pharmaceutique après méthylation de la fonction phénolique.

Le dichloro-2,4 nitro-6 phénol peut servir notamment comme inhibiteur d'enzyme.

Les monochloronitrophénols peuvent être utilisés comme fongicides ou comme intermédiaires pour la préparation de fongicides.

La chloration d'un nitrophénol est cependant difficile, car la présence de la fonction $NO_2$ sur le cycle benzènique désactive fortement la molécule.

Il a maintenant été trouvé que l'on peut améliorer la chloration des nitrophénols en monochloronitrophénols ou dichloronitrophénols en opérant en présence d'une amine.

Plus précisément, la présente invention consiste en un procédé de chloration par le chlore gazeux d'orthonitrophénol ou de paranitrophénol, caractérisé en ce que l'on opère à l'état fondu et en présence d'une quantité efficace d'une amine primaire, secondaire ou tertiaire.

Par amine on entend dans le présent texte tout composé, liquide ou solide dans les conditions opératoires du procédé, comportant une ou plusieurs fonctions amine.

Un tel composé peut également comporter une ou plusieurs autres fonctions chimiques, telles que par exemple la fonction hydroxyle, la fonction acide carboxylique, la fonction ester d'acide carboxylique, la fonction amide ou la fonction imine.

Il est bien évident que les amines utilisées peuvent être introduites également sous la forme de leurs sels et plus particulièrement de leurs chlorhydrates respectifs.

Dans le présent texte, le terme "amine" englobe également l'ammoniac ainsi que les sels et notamment les chlorhydrates d'amines.

Les amines utilisées comme catalyseur dans le présent procédé sont plus particulièrement les amines de formule générale (I) :

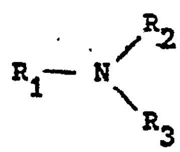

(I)

dans laquelle :

- $R_1$, $R_2$ et $R_3$, identiques ou différents, représentent :

. un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther -O- ou fonctions hydroxyle, amine, acide carboxylique, ester d'acide carboxylique, amide ou imine ;

. un radical phényle, un radical cyclohexyle, un radical cycloheptyle ou un radical cyclopentyle ;

. un radical phénylalkyle, cyclohexylalkyle, cycloheptylalkyle ou cyclopentylalkyle dont la partie alkyle comporte 1 à 4 atomes de carbone ;

. un atome d'hydrogène ;

- $R_1$ peut représenter un groupement $NH_2$ ;

- $R_2$ et $R_3$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone ;

- $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;

- $R_1$, $R_2$ et $R_3$ forment ensemble et avec l'atome d'azote un hétérocycle insaturé éventuellement substitué par un ou deux groupements méthyle ou éthyle ;

- $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

A titre d'exemples illustratifs, on peut citer comme amines de formule (I) :

- l'ammoniac ;

- des amines primaires telles que la n-propylamine, l'isopropylamine, l'isobutylamine, la n-butylamine, la tertiobutylamine, la n-pentylamine, la méthyl-2 butylamine, la méthyl-3 butylamine, la n-hexylamine, l'éthyl-2 hexylamine, l'aniline, la laurylamine, la cyclohexylamine, la cyclopentylamine, la benzylamine, la guanidine, l'acétamidine, l'ester éthylique de la glycine, l'éthanolamine, l'éthylènediamine, l'hexaméthylènediamine, la N-aminoéthylpyrrolidine, la pyrazoline, la lysine, la N-aminomorpholine, la N-aminopipéridine ;

- des amines secondaires telles que la dibutylamine, la dipropylamine, la méthylpropylamine, la méthylbu-

tylamine, la méthylisobutylamine, la méthyltertiobutylamine, la méthylbenzylamine, la ditertiobutylamine, la méthyl-1 cyclopentylamine, la méthyl-1 cyclohexylamine, la dicyclohexylamine, la morpholine, l'imidazole, la pyrrolidine, l'imidazolidine, la pipérazine, l'indole ;

- des amines tertiaires telles que la triéthylamine, la tributylamine, la pyridine, la tris(dioxa-3,6 heptyl)amine, le diaza-1,8 bicyclo(5,4,0)undécène-7.

On peut utiliser également des composés aminés comme l'hydrazine ou ses dérivés, notamment les dérivés obtenus par substitution d'un ou de deux atomes d'hydrogène par des radicaux alkyles, aryles, cycloaliphatiques ou hétérocycliques.

La quantité d'amine utilisée dans le procédé peut varier dans de très larges limites.

Habituellement elle représente en poids par rapport au poids du nitrophénol de 0,005 % à 10 %. Préférentiellement on mettra en oeuvre de 0,015 % à 5 % en poids d'amine par rapport au nitrophénol, afin d'avoir une efficacité suffisante, tout en n'ayant pas une quantité excessive d'amine.

Parmi les amines de formule générale (I), on préfère dans le cadre du présent procédé, les amines primaires ou secondaires de formule (II) :

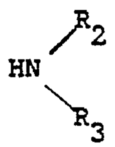 (II)

dans laquelle :

- $R_2$ ou $R_3$ représente un atome d'hydrogène ;
- $R_2$ et $R_3$, identiques ou différents, représentent :
. un radical alkyle linéaire ayant 1 à 10 atomes de carbone ;
. un radical alkyle secondaire ayant 3 à 10 atomes de carbone ;
. un radical alkyle tertiaire ayant 4 à 10 atomes de carbone ;
. un radical cyclohexyle ou cyclopentyle ;
. un radical phényle ;
. un radical benzyle ou phénéthyle ;
- $R_2$ et $R_3$ forment ensemble, avec l'atome d'azote et avec un autre atome d'azote et/ou d'oxygène, un hétérocycle saturé ou comportant une ou plusieurs insaturations ;
- $R_2$ et/ou $R_3$ comportent un ou plusieurs fonctions amine, hydroxyle ou ester d'acide carboxylique.

A titre d'exemples d'amines primaires de formule générale (II), on peut citer la n-propylamine, l'isopropylamine, la n-butylamine, l'isobutylamine, la tertiobutylamine, la n-pentylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine et l'éthanolamine.

En ce qui concerne plus particulièrement les amines secondaires de formule générale (II), on préfère encore plus spécialement celles pour lesquelles au moins un des symboles $R_2$ et $R_3$ et de préférence les deux symboles $R_2$ et $R_3$ représentent :

. un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5 ;
. un radical cyclohexyle ou cyclopentyle ;

et celles pour lesquelles $R_2$ et $R_3$ forment avec l'atome d'azote un hétérocycle comportant éventuellement un autre atome d'azote ou un atome d'oxygène.

A titre d'exemples de telles amines secondaires, on peut citer la diisopropylamine, la diisobutylamine, la dicyclohexylamine, la morpholine, l'imidazole.

La catalyse par les amines de la chloration des nitrophénols permet une meilleure fixation du chlore sur la molécule de nitrophénol et plus particulièrement favorise la fixation du deuxième atome de chlore.

Alors que sans amine il est nécessaire d'avoir un important excès de chlore dès le stade de la première chloration, cet excès est beaucoup plus faible en présence d'amine.

La quantité de chlore utilisée dans le présent procédé dépend essentiellement du stade de chloration souhaité.

Si l'on désire arrêter la chloration au stade du monochloronitrophénol, il n'est pas nécessaire d'avoir un rapport molaire chlore introduit/nitrophénol chargé très supérieur à la stoechiométrie. Généralement ce rapport molaire sera compris entre 1 et 2.

Si l'on désire former du dichloronitrophénol, il est préférable d'avoir un rapport molaire chlore introduit/nitrophénol chargé compris entre 2 et 10 et plus particulièrement compris entre 3 et 6.

En pratique, le plus souvent, le chlore est introduit par barbotage dans le milieu réactionnel. La pression dans l'appareillage est donc sensiblement égale ou légèrement supérieure à la pression atmosphérique.

Le chlore peut être utilisé seul ou être dilué par un gaz inerte, tel que l'azote par exemple. La présence d'un gaz inerte permet, si nécessaire, d'augmenter le débit gazeux sans augmenter corrélativement la quantité de chlore introduite en un temps donné.

Le chlore gazeux utilisé dans le présent procédé peut également être formé in situ, à partir d'acide chlorhydrique, par addition d'un composé oxydant, tel que par exemple le peroxyde d'hydrogène.

Un autre avantage très intéressant de la chloration des nitrophénols en présence d'une amine consiste dans la modification de la régiosélectivité de la réaction.

En effet lorsque l'on chlore l'orthonitrophénol sans amine, on forme au premier stade de la chloration une importante majorité de chloro-4 nitro-2 phénol par rapport au chloro-2 nitro-6 phénol.

Lorsque l'on opère en présence d'une amine, le chloro-2 nitro-6 phénol devient majoritaire. On dispose donc ainsi d'un procédé sélectif d'obtention du chloro-2 nitro-6 phénol à partir de l'orthochlorophénol.

La température à laquelle est mis en œuvre le procédé de l'invention est généralement inférieure ou égale à 200°C. La limite inférieure n'est pas critique. Elle est conditionnée par la nécessité d'avoir un mélange réactionnel liquide.

Les températures préférées seront comprises entre le point de fusion du nitrophénol engagé et 150°C.

Le procédé de l'invention peut être réalisé de manière continue ou discontinue.

Les exemples qui suivent illustrent l'invention.

EXEMPLE 1

Dans un ballon en verre de 250 $cm^3$ équipé d'une agitation mécanique, d'un tube plongeant pour l'injection de chlore, d'un réfrigérant ascendant et d'une gaine thermométrique, on charge:
- paranitrophénol: 34,78 g (0,250 mole)
- diisopropylamine: 0,34 g (1% en poids par rapport au paranitrophénol).

On chauffe sous agitation à 120°C à l'aide d'un bain thermostaté, puis on envoie du chlore gazeux par le tube plongeant avec un débit de 5 l/h. On maintient la température à 120°C pendant toute la chloration.

Après 2 h 15 min de chloration, ce qui représente l'introduction de 0,5 mole de chlore, on dose par chromatographie liquide à haute performance (CLHP).la composition du mélange réactionnel final.

On obtient les résultats suivants:
– Taux de transformation (TT) du paranitrophénol: 100 %
– Rendement (RT) en chloro-2 nitro-4 phénol: 76,3 %
– RT en dichloro-2,6 nitro-4 phénol: 23,7%

ESSAI COMPARATIF A

On répète l'exemple 1 dans les mêmes conditions mais sans charger d'amine. On obtient les résultats suivants :
- Taux de transformation (TT) du paranitrophénol : 98,8 %
- Rendement (RT) en chloro-2 nitro-4 phénol : 86,2 %
- RT en dichloro-2,6 nitro-4 phénol : 13,8 %

EXEMPLE 2 et ESSAI COMPARATIF B

Dans l'appareillage décrit pour l'exemple 1, on charge :
- orthonitrophénol (ONP) : 34,78 g (0,250 mole)
- diisopropylamine : 0,34 g (1 % en poids par rapport à l'orthonitrophénol).

On chauffe sous agitation à 75°C, puis on envoie le chlore gazeux avec un débit de 5 litres/heure.

On maintient la température à 75°C pendant toute la chloration.

Après 1 h 07 min de chloration, ce qui représente l'introduction de 0,25 mole de chlore, on effectue un prélèvement du mélange réactionnel que l'on dose par CLHP et par résonance magnétique nucléaire (RMN).

On poursuit la chloration. Après 2 h 15 min (ce qui représente l'introduction de 0,50 mole de chlore) on arrête l'essai et on dose le mélange final par CLHP et RMN.

On répète le même essai dans les mêmes conditions, mais sans amine.

Le tableau ci-après rassemble les résultats obtenus pour l'exemple 2 et l'essai B, à chacun des deux stades de la chloration.

| | | EXEMPLE 2 | ESSAI COMPARATIF B |
|---|---|---|---|
| PREMIER STADE DE CHLORATION | TT de l'orthonitrophénol | 78 % | 41 % |
| | RT en chloro-2 nitro-6 phénol | 66 % | 10 % |
| | RT en chloro-4 nitro-2 phénol | 29 % | 90 % |
| | Rapport dérivé ortho / dérivé para (*) | 2,3 | 0,11 |
| | RT en dichloro-2,4 nitro-6 phénol | 5 % | 0 % |
| DEUXIEME STADE DE CHLORATION | TT de l'orthonitrophénol | 100 % | 88 % |
| | RT en chloro-2 nitro-6 phénol | 49 % | 14 % |
| | RT en chloro-4 nitro-2 phénol | 21 % | 84 % |
| | Rapport dérivé ortho / dérivé para (*) | 2,3 | 0,17 |
| | RT en dichloro-2,4 nitro-6 phénol | 30 % | 2 % |

(*) rapport molaire chloro-2 nitro-6 phénol (dérivé ortho)/chloro-4 nitro-2 phénol (dérivé para).

**Revendications**

1°) Procédé de chloration par le chlore gazeux d'orthonitrophénol ou de paranitrophénol, caractérisé en ce que l'on opère à l'état fondu et en présence d'une quantité efficace d'une amine primaire, secondaire ou tertiaire.

2°) Procédé selon la revendication 1, caractérisé en ce que l'amine utilisée est une amine de formule générale (I) :

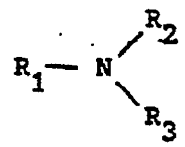
(I)

dans laquelle :
- $R_1$, $R_2$ et $R_3$, identiques ou différents, représentent :
. un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther -O- ou fonctions hydroxyle, amine, acide carboxylique, ester d'acide carboxylique, amide ou imine ;
. un radical phényle, un radical cyclohexyle, un radical cycloheptyle ou un radical cyclopentyle ;
. un radical phénylalkyle, cyclohexylalkyle, cycloheptylalkyle ou cyclopentylalkyle dont la partie alkyle comporte 1 à 4 atomes de carbone ;
. un atome d'hydrogène ;
- $R_1$ peut représenter un groupement $NH_2$ ;
- $R_2$ et $R_3$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone ;
- $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;
- $R_1$, $R_2$ et $R_3$ forment ensemble et avec l'atome d'azote un hétérocycle insaturé éventuellement substitué par un ou deux groupements méthyle ou éthyle ;
- $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

3°) Procédé selon l'une des revendications 1 ou 2, caractérisé en ce que la quantité d'amine utilisée représente en poids par rapport au poids du nitrophénol de 0,005 % à 10 % et de préférence de 0,015 % à 5 %.

4°) Procédé selon l'une des revendications 1 à 3, caractérisé en ce que l'on utilise des amines primaires ou secondaires de formule (II) :

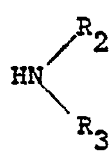
(II)

dans laquelle :
- $R_2$ ou $R_3$ représente un atome d'hydrogène ;
- $R_2$ et $R_3$, identiques ou différents, représentent :
. un radical alkyle linéaire ayant 1 à 10 atomes de carbone ;
. un radical alkyle secondaire ayant 3 à 10 atomes de carbone ;
. un radical alkyle tertiaire ayant 4 à 10 atomes de carbone ;
. un radical cyclohexyle ou cyclopentyle ;
. un radical phényle ;
. un radical benzyle ou phénéthyle ;
- $R_2$ et $R_3$ forment ensemble, avec l'atome d'azote et avec un autre atome d'azote et/ou d'oxygène, un hétérocycle saturé ou comportant une ou plusieurs insaturations ;
– $R_2$ et/ou $R_3$ comportent une ou plusieurs fonctions amine, hydroxyle ou ester d'acide carboxylique.

5. Procédé selon l'une des revendications 1 à 4, caractérisé en ce que l'on utilise des amines secondaires de formule générale (II)

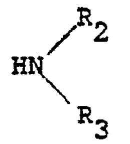

(II)

dans laquelle au moins un des symboles $R_2$ et $R_3$ et de préférence les deux symboles $R_2$ et $R_3$ représentent:
– un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5;
– un radical cyclohexyle ou cyclopentyle;
ou des amines secondaires de formule générale (II) dans laquelle $R_2$ et $R_3$ forment avec l'atome d'azote un hétérocycle comportant éventuellement un autre atome d'azote ou un atome d'oxygène.

6. Procédé selon l'une des revendications 1 à 5, caractérisé en ce que l'on utilise comme amine la diisopropylamine, la diisobutylamine, la dibutylamine, la dicyclohexylamine, la morpholine, l'imidazole.

7. Procédé selon l'une des revendications 1 à 5, caractérisé en ce que l'on utilise comme amine la n-propylamine, l'isopropylamine, la n-butylamine, l'isobutylamine, la tertiobutylamine, la n-pentylamine, la méthyl-2 butylamine, la méthyl-3 butylamine, la n-hexylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine ou l'éthanolamine.

8. Procédé selon l'une des revendications 1 à 7, caractérisé en ce que l'on opère à une température égale ou inférieure à 200°C et de préférence comprise entre le point de fusion des nitrophénols et 150°C.

9. Procédé de préparation du chloro-2 nitro-6 phénol par chloratoin de l'orthonitrophénol selon l'une des revendications 1 à 8.

**Claims**

1. Process for the chlorination of ortho-nitrophenol or para-nitrophenol with gaseous chlorine, characterized in that the reaction is performed in the molten state and in the presence of an effective quantity of a primary, secondary or tertiary amine.

2. Process according to claim 1, characterized in that the amine used is an amine of general formula (I):

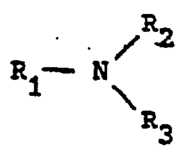

(I)

in which:
$R_1$, $R_2$ and $R_3$, which may be identical or different, denote:
– a linear alkyl radical having from 1 to 12 carbon atoms, a secondary alkyl radical having 3 to 12 carbon atoms or a tertiary alkyl radical having 4 to 12 carbon atoms, it being possible for these alkyl radicals to contain one or two –O– ether groups or hydroxyl, amine, carboxylic acid, carboxylic acid ester, amide or imine groups;
– a phenyl radical, a cyclohexyl radical, a cycloheptyl radical or a cyclopentyl radical;
– a phenylalkyl, cyclohexylalkyl, cycloheptylalkyl or cyclopentylalkyl radical in which the alkyl portion contains 1 to 4 carbon atoms;
– a hydrogen atom;
$R_1$ can denote an $NH_2$ group;
$R_2$ and $R_3$ form, together and with the nitrogen atom, a saturated heterocycle or a heterocycle containing one or more double bonds, optoinally substituted with one or more alkyl groups having 1 to 4 carbon atoms;
$R_2$ and $R_3$ or $R_1$, and $R_3$ form, together with the nitrogen atom and with one or more nitrogen and/or oxygen and/or sulphur atoms, a saturated or unsaturated heterocycle optionally substituted with one or more alkyl groups having 1 to 4 carbon atoms;
$R_1$, $R_2$ and $R_3$ form, together and with the nitrogen atom, an unsaturated heterocycle optionally substituted with one or two methyl or ethyl groups;
$R_2$ and $R_3$ or $R_1$, $R_2$ and $R_3$ form, together with the nitrogen atom and optionally with one or more nitrogen and/or oxygen and/or sulphur atoms, a saturated or unsaturated polycyclic compound optionally substituted with one or more alkyl groups having 1 to 4 carbon atoms.

3. Process according to one of claims 1 and 2, characterized in that the quantity of amine used represents from 0.005% to 10%, and preferably from 0.015% to 5%, by weight relative to the weight of the nitrophenol.

4. Process according to one of claims 1 to 3, characterized in that use is made of primary or secondary amines of formula (II):

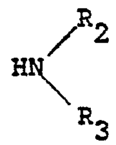 (II)

in which:
$R_2$ or $R_3$ denotes a hydrogen atom;
$R_2$ and $R_3$, which may be identical or different, denote:
– a linear alkyl radical having 1 to 10 carbon atoms;
– a secondary alkyl radical having 3 to 10 carbon atoms;
– a tertiary alkyl radical having 4 to 10 carbon atoms;
– a cyclohexyl or cyclopentyl radical;
– a phenyl radical;
– a benzyl or phenethyl radical;
$R_2$ and $R_3$ form, together with the nitrogen atom and with another nitrogen and/or oxygen atom, a saturated heterocycle or a heterocycle containing one or more unsaturated bonds;
$R_2$ and/or $R_3$ contain one or more amine, hydroxyl or carboxylic acid ester groups.

5. Process according to one of claims 1 to 4, characterized in that use is made of secondary amines of general formula (II):

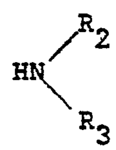 (II)

in which at least one of the symbols $R_2$ and $R_3$, and preferably both symbols $R_2$ and $R_3$, denote:
– a secondary alkyl radical having from 3 to 10 carbon atoms, such as isopropyl, 2-butyl, 2-pentyl, 3-pentyl, 2-hexyl, 3-hexyl, 2-heptyl, 3-heptyl, 4-heptyl, 2-octyl, 3-octyl, 4-octyl, 2-nonyl, 3-nonyl, 4-nonyl, 5-nonyl, 2-decyl, 3-decyl, 4-decyl and 5-decyl;
– a cyclohexyl or cyclopentyl radical; or secondary amines of general formula (II) in which $R_2$ and $R_3$ from, with the nitrogen atom, a heterocycle optionally containing one other nitrogen atom or an oxygen atom.

6. Process according to one of claims 1 to 5, characterized in that diisopropylamine, diisobutylamine, dibutylamine, dicyclohexylamine, morpholine or imidazole is used as an amine.

7. Process according to one of claims 1 to 5, characterized in that n-propylamine, isopropylamine, n-butylamine, isobutylamine, tert-butylamine, n-pentylamine, 2-methylbutylamine, 3-methylbutylamine, n-hexylamine, 2-methylpentylamine, 3-methylpentylamine, 2-ethylhexylamine, laurylamine, cyclohexylamine, cyclopentylamine, benzylamine, glycine ethyl ester or ethanolamine is used as an amine.

8. Process according to one of claims 1 to 7, characterized in that the reaction is performed at a temperature equal to or below 200°C, and preferably between the melting point of the nitrophenols and 150°C.

9. Process for prepearing 2-chloro-6-nitrophenol by the chlorination of orthonitrophenol according to one of claims 1 to 8.

## Patentansprüche

1. Verfahren zur Chlorierung von Orthonitrophenol oder Paranitrophenol mit gasförmigem Chlor, dadurch gekennzeichnet, daß man in geschmolzenem Zustand und in Gegenwart einer wirksamen Menge eines primären, sekundären oder tertiären Amins arbeitet.

2. Verfahren gemäß Anspruch 1, dadurch gekennzeichnet, daß das verwendete Amin ein Amin der allgemeinen Formel (I)

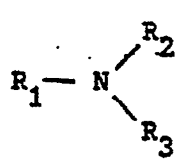 (I)

ist, worin

$R_1$, $R_2$ und $R_3$, die gleich oder voneinander verschieden sind, bedeuten:
einen linearen Alkylrest mit 1 bis 12 Kohlenstoffatomen, einen sekundären Alkylrest mit 3 bis 12 Kohlenstoffatomen oder einen tertiären Alkylrest mit 4 bis 12 Kohlenstoffatomen, wobei diese Alkylreste eine oder zwei Etherfunktionen –O– oder Hydroxyl-, Amin-, Carbonsäure-, Carbonsäureester, Amid- oder Imin-Funktionen aufweisen können;
einen Phenylrest, einen Cyclohexylrest, einen Cycloheptylrest oder einen Cyclopentylrest;
einen Phenylalkyl-, Cyclohexylalkyl-, Cycloheptylalkyl- oder Cyclopentylalkylrest, deren Alkylteil 1 bis 4 Kohlenstoffatome besitzt;
ein Wasserstoffatom;
– $R_1$ eine $NH_2$-Gruppe bedeuten kann;
– $R_2$ und $R_3$ gemeinsam mit dem Stickstoffatom einen Heterocyclus bilden, der gesättigt ist oder eine oder mehrere Doppelbindungen aufweist und gegebenenfalls durch eine oder mehrere Alkylgruppen mit 1 bis 4 Kohlenstoffatomen substituiert ist;
– $R_2$ und $R_3$ oder $R_1$, $R_2$ und $R_3$ gemeinsam mit dem Stickstoffatom und mit einem oder mehreren Stickstoff- und/oder Sauerstoff- und/oder Schwefelatomen einen gesättigten oder ungesättigten Heterocyclus bilden, der gegebenenfalls durch einen oder mehrere Alkylreste mit 1 bis 4 Kohlenstoffatomen substituiert ist;
– $R_1$, $R_2$ und $R_3$ gemeinsam mit dem Stickstoffatom einen ungesättigten Heterocyclus bilden, der gegebenenfalls durch eine oder zwei Methyl- oder Ethylgruppen substituiert ist;
– $R_2$ und $R_3$ oder $R_1$, $R_2$ und $R_3$ gemeinsam mit dem Stickstoffatom und gegebenenfalls mit einem oder mehreren Stickstoff- und/oder Sauerstoff- und/oder Schwefelatomen eine gesättigte oder ungesättigte, polycyclische Verbindung bilden, die gegebenenfalls durch eine oder mehrere Alkylgruppen mit 1 bis 4 Kohlenstoffatomen substituiert ist.

3. Verfahren gemäß einem der Ansprüche 1 oder 2, dadurch gekennzeichnet, daß die Menge an verwendetem Amin, ausgedrückt durch das Gewicht in bezug auf das Gewicht des Nitrophenols, 0,005 bis 10%, vorzugsweise 0,015 bis 5%, beträgt.

4. Verfahren gemäß einem der Ansprüche 1 bis 3, dadurch gekennzeichnet, daß man primäre oder sekundäre Amine der Formel (II)

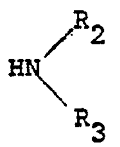 (II)

verwendet, worin
$R_2$ oder $R_3$ für ein Wasserstoffatom steht;
$R_2$ und $R_3$, die gleich oder voneinander verschieden sind bedeuten:
– ein lineare Alkylgruppe mit 1 bis 10 Kohlenstoffatomen;
– eine sekundäre Alkylgruppe mit 3 bis 10 Kohlenstoffatomen;
– eine tertiäre Alkylgruppe mit 4 bis 10 Kohlenstoffatomen;
– einen Cyclohexyl- oder Cyclopentylrest;
– einen Phenylrest;
– einen Benzyl- oder Phenethylrest;
$R^2$ und $R_3$ gemeinsam mti dem Stickstoffatom und mit einem weiteren Stickstoff- und/oder Sauerstoffatom einen Heterocyclus bilden, der gesätigt ist oder eine oder mehrere Unsättigungen aufweist;
$R_2$ und/oder $R_3$ eine oder mehrere Amin-, Hydroxyl- oder Carbonsäureester-Funktionen aufweisen.

5. Verfahren gemäß einem der Ansprüche 1 bis 4, dadurch gekennzeichnet, daß man sekundäre Amine der allgemeinen Formel (II)

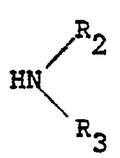 (II)

verwendet, worin zumindest eines der Symbole $R_2$ und $R_3$ und vorzugsweise beide Symbole $R_2$ und $R_3$ bedeuten:
– einen sekundären Alkylrest mit 3 bis 10 Kohlenstoffatomen, wie Isopropyl, 2-Butyl, 2-Pentyl, 3-Pentyl, 2-Hexyl, 3-Hexyl, 2-Heptyl, 3-Heptyl, 4-Heptyl, 2-Octyl, 3-Octyl, 4-Octyl, 2-Nonyl, 3-Nonyl, 4-Nonyl, 5-Nonyl, 2-Decyl, 3-Decyl, 4-Decyl und 5-Decyl;
– einen diejenigen sekundären Amine der allgemeinen Formel (II), worin $R_2$ und $R_3$ mit dem Stickstoffatom einen Heterocyclus bilden, der gegebenenfalls ein weiteres Stickstoffatom oder ein Sauerstoffatom aufweist.

6. Verfahren gemäß einem der Ansprüche 1 bis 5, dadurch gekennzeichnet, daß man als Amin Diisopropylamin, Diisobutylamin, Dibutylamin, Dicyclohexylamin, Morpholin oder Imidazol verwendet.

7. Verfahren gemäß einem der Ansprüche 1 bis 5, dadurch gekennzeichnet, daß man als Amin n-Propylamin, Isopropylamin, n-Butylamin, Isobutylamin, tert.-Butylamin, n-Pentylamin, 2-Methylbutylamin, 3-Methylbutylamin, n-Hexylamin, 2-Methylpentylamin, 3-Methylpentylamin, 2-Ethylhexylamin, Laurylamin, Cyclohexylamin, Cyclopentylamin, Benzylamin, Glycinethylester oder Ethanolamin einsetzt.

8. Verfahren gemäß einem der Ansprüche 1 bis 7, dadurch gekennzeichnet, daß man bei einer Temperatur gleich oder niedriger als 200°C und vorzugsweise zwischen dem Schmelzpunkt der Nitrophenole und 150°C arbeitet.

9. Verfahren zur Herstellung von 2-Chlor-6-nitrophenol durch Chlorierung von Orthonitrophenol gemäß einem der Ansprüche 1 bis 8.